# FASCICULE DE BREVET EUROPEEN

(11) **EP 3 714 865 B1**
(45) Date de publication et mention de la délivrance du brevet: **02.08.2023**
(21) Numéro de dépôt: 20164825.0
(22) Date de dépôt: 23.03.2020
(51) Int. Cl.: A61K 8/37, A61K 8/39, A61K 8/42, A61K 8/44, A61K 8/49, A61Q 17/04

(54) **COMPOSITION COSMETIQUE SOLAIRE A HAUTE TENEUR EN FILTRES SOLAIRES DERIVES DE TRIAZINE**
KOSMETISCHE SONNENSCHUTZZUSAMMENSETZUNG MIT HOHEM SONNENSCHUTZFILTERANTEIL AUF TRIAZINBASIS
COSMETIC COMPOSITION WITH HIGH CONTENT OF TRIAZINE DERIVATIVE SOLAR FILTERS

(30) Priorité: 29.03.2019 FR 1903400
(43) Date de publication de la demande: 30.09.2020
(60) Demande divisionnaire: 23181935.0
(73) Titulaire: Thorel, Jean-Noël, 75014 Paris (FR); NAOS Institute of Life Science, 13290 Aix-en-Provence (FR)
(72) Inventeur: THOREL, Jean-Noël, 75014 PARIS (FR)
(74) Mandataire: Cabinet Laurent & Charras

(56) Documents cités:
- EP-A1- 2 561 853
- DE-A1-102007 017 436
- DATABASE GNPD [Online] MINTEL; 13 février 2019 (2019-02-13), anonymous: "Sun Care Oil Mist for Body & Hair SPF 30", XP055656407, extrait de www.gnpd.com Database accession no. 6344721

## Description

### DOMAINE DE L'INVENTION

La présente invention concerne une composition, avantageusement cosmétique ou dermatologique, à haute teneur en filtres solaires organiques dérivés de triazine et son utilisation pour protéger la peau du rayonnement ultraviolet.

### ETAT ANTÉRIEUR DE LA TECHNIQUE

Le rayonnement ultraviolet (UV) se compose de lumière de longueur d'onde comprise entre 100 nm et 400 nm, traditionnellement répartie en 3 sous-groupes : les UV-A (400-315 nm), les UV-B (315-280 nm) et les UV-C (280-100 nm). Les UV-C, de courte longueur d'onde, sont les UV les plus énergétiques et les plus nocifs. Ils sont filtrés par la couche d'ozone de l'atmosphère et n'atteignent pas la surface de la Terre en quantité considérable. La plupart des dégâts sur la peau restent causés par les UV-A et les UV-B.

Les UV-B induisent la production du pigment naturel de la peau appelé mélanine. Ce qui est à l'origine du phénomène communément appelé bronzage. Les UV-B stimulent également les cellules pour qu'elles produisent un épiderme plus épais. Ces réactions constituent un mécanisme de défense de l'organisme contre le rayonnement UV.

L'énergie importante des UV-B génère des désordres moléculaires (altération de l'ADN et dommages aux protéines, notamment leur carbonylation) qui, à long terme, saturent et enrayent le système de réparation de 1'ADN nucléaire. Cela entraîne des mutations permanentes dans le génome des cellules atteintes par les UV-B, qui sont à l'origine de cancers cutanés. Il s'agit alors d'une toxicité directe des UV-B. Quant aux UV-A, il est connu qu'ils pénètrent dans les couches profondes de la peau, où ils produisent des effets délétères notamment au sein du tissu conjonctif et sur les vaisseaux sanguins. Ils sont notamment à l'origine du phénomène nommé héliodermie, c'est-à-dire le vieillissement actinique prématuré de la peau.

De surcroit, bien que les UV-B soient les principaux responsables des cancers cutanés, les UV-A ont également une contribution indirecte à ce type d'atteinte. En effet, les UV-A et les UV-B sont à l'origine de la production de radicaux libres, notamment les ROS (*Reactive Oxygen Species* ou espèces réactives de l'oxygène (ERO)). Il s'agit de molécules très instables ayant une demi-vie très courte, de l'ordre de la nanoseconde à la milliseconde. Les ROS sont capables d'endommager les structures intracellulaires (ADN, membranes, protéines intracellulaires...) ainsi que les structures extracellulaires (les composants de la matrice extracellulaire comme les fibres de collagène...). Les ROS exercent également une action nocive indirecte, en provoquant l'oxydation des lipides membranaires, ce qui induit la formation des espèces réactives carbonylés qui contribuent à amplifier les dégâts tissulaires et cellulaires. Les mêmes dommages sont également causés et même amplifiés par la pollution environnementale, qui génère également des radicaux libres responsables du vieillissement cutané.

Il est donc nécessaire de lutter contre les effets nocifs du rayonnement UV, notamment UV-A et UV-B, en tenant compte des différences interindividuelles telles que le type de peau (phototype) et les temps d'exposition au soleil.

Une stratégie de lutte contre les dommages causés par le soleil sur la peau est la protection externe. Elle s'effectue en filtrant le rayonnement UV ou en occultant les zones exposées au soleil. Le recouvrement des zones exposées assure une photoprotection totale, mais n'est pas toujours possible ou pratique. Pour cette raison, des compositions solaires ont été développées afin d'assurer une photoprotection des zones sujettes au rayonnement UV. Ces compositions se présentent sous la forme de lotion, huile, émulsion de type huile-dans-eau ou eau-dans-huile, mousse, gel, stick ou spray. Elles contiennent un support cosmétiquement acceptable et un ou plusieurs filtres solaires chimiques (ou organiques) ou écrans minéraux à des concentrations diverses.

Les associations de filtres solaires chimiques et/ou écrans minéraux, qui ont des spectres d'absorption différents, et les quantités mises en oeuvre, sont sélectionnées en fonction du niveau de protection solaire recherché et/ou de la zone du corps à protéger.

Les écrans solaires minéraux, parmi lesquels on peut citer les oxydes de zinc et les dioxydes de titane, protègent des UV en reflétant la lumière solaire. Ils agissent donc comme des miroirs localisés à la surface de la peau. Les écrans minéraux, du moins sous la formule micrométrique, sont sûrs pour l'utilisation humaine et ne comportent aucun risque pour la santé.

Cependant, ils présentent d'autres inconvénients :
- les écrans minéraux ont tendance à dessécher la peau et à s'étaler avec difficulté, en laissant des trainées blanches sur la peau qui peuvent se révéler très inesthétiques ;
- l'appréciation galénique des compositions à base d'écrans minéraux est souvent médiocre, notamment dans les compositions exemptes de silicones.

Ces caractéristiques techniques des écrans minéraux peuvent limiter l'observance des utilisateurs de produits à base de ce système de filtration de la lumière UV.

Les filtres chimiques sont des composés organiques qui absorbent la lumière en créant sur la peau une couche filtrante qui neutralise les rayons UV. Ainsi, lorsque la molécule de filtre solaire reçoit une radiation UV, elle passe dans un état excité en absorbant l'énergie du rayonnement puis retourne dans son état stable. Par ce mécanisme, la radiation lumineuse est convertie en chaleur et dissipée. Les filtres chimiques agissent donc de la même façon que la mélanine, le pigment photoprotecteur naturel de la peau.

Les filtres solaires chimiques présentent plusieurs aspects avantageux. Ils sont, en majorité, lipophiles et se prêtent donc à la formulation de produits anhydres hautement résistant à l'eau. Ils agissent de façon synergique en couvrant des plages de longueur d'onde différentes, ce qui permet d'obtenir une protection uniforme dans les UV-B et UV-A voire même jusqu'au rayonnement visible à haute énergie. Les filtres chimiques peuvent également se stabiliser, et notamment se photostabiliser réciproquement, ce qui peut contribuer à assurer la rémanence de la photoprotection.

Ces caractéristiques des filtres organiques ont été à la base de leur succès : la plupart des produits solaires, notamment les produits solaires à haute protection caractérisés par un facteur de protection solaire (FPS ou SPF pour la dénomination anglaise « Sun Protection Factor ») supérieur à 30 sont formulés en employant des associations de filtres organiques, éventuellement complémentées par des quantités mineures d'écrans minéraux.

En Europe, l'ensemble des filtres organiques ou écrans minéraux autorisés par la réglementation ainsi que leurs concentrations limites d'utilisation sont inscrits dans une liste (Annexe VI de la Directive européenne sur les produits cosmétiques). Il s'agit d'une liste regroupant à ce jour une trentaine de composés selon leur désignation INCI, parmi dans laquelle l'industrie cosmétique doit puiser afin de formuler les produits de protection solaire. Toutefois, cette liste n'est pas figée et évolue constamment en fonction des résultats des recherches scientifiques. En effet, plusieurs études scientifiques ont démontré qu'une partie des filtres organiques de cette liste pourraient être à l'origine de risque pour la santé humaine et l'environnement.

En particulier, des résultats scientifiques mettent en doute l'innocuité de la benzophénone-4 ou encore de la benzophénone-3, également appelée oxybenzone.

Plusieurs études ont ainsi montré que les filtres solaires mentionnés précédemment sont susceptibles d'être des perturbateurs endocriniens qui interfèrent avec le développement embryonnaire chez le poisson-zèbre (ou *Danio rerio),* notamment au niveau de la formation de la glande thyroïdienne, des gonades, du cerveau ou encore du foie (Kinnberg *et al.* 2015 ; Zucchi *et al.* 2011).

Un autre filtre solaire organique fortement suspecté d'être un perturbateur endocrinien est l'octyl méthoxycinnamate (ou l'éthylhexyl méthoxycinnamate ou octinoxate). Ce filtre est pourtant encore présent dans la plupart des produits solaires disponibles à ce jour. Cette molécule perturbe l'axe hypothalamo-hypophysaire chez le rat (Szwarcfarb *et al.* 2008) et provoque des atteintes des fonctions reproductives et neurologiques des descendants des femelles exposées (Axelstad *et al.* 2011).

Le 3-méthylbenzylidène camphor et le 4-méthylbenzylidène camphor représentent deux autres exemples de filtres solaires largement utilisés dans le passé qui sont maintenant sujets à controverse en raison de leurs effets sur le système endocrinien. Ils seraient à l'origine d'atteintes des organes reproducteurs, d'une perturbation du comportement sexuel et d'une modification de l'expression génique associée chez les descendants des rats femelles exposées (Schlumpf *et al.* 2008). En outre, il serait établi que cette activité de perturbation du système endocrinien s'appliquerait également à l'homme (Petersen *et al.* 2007). Pour cette raison, l'utilisation du 3-méthylbenzylidène camphor, en particulier, dans les produits cosmétiques est désormais interdite en France.

Quant au 4-méthylbenzylidène camphor, il pourrait également être interdit dans un futur proche au vue des résultats des recherches scientifiques.

Un certain nombre d'autres filtres solaires chimiques, comme l'octocrylène, l'isoamyl méthoxycinnamate, ou encore l'octyl diméthyl PABA, pourraient également être interdits dans les formulations cosmétiques solaires, en raison de données scientifiques, pour l'instant limitées à des études *in vitro* ou sur des modèles d'animaux invertébrés, montrent qu'ils pourraient également avoir des effets néfastes sur le système endocrinien (Schlumpf *et al.* 2001 ; Rehfeld *et al.* 2016 ; Ozáez *et al.* 2016).

En parallèle des éventuels effets chez l'homme, une partie de ces molécules est retrouvés dans le milieu naturel, notamment dans l'eau de baignade ou dans les eaux usées évacuées au cours des douches. Cette pollution des eaux associée aux filtres UV se répercute inévitablement sur toute la chaine alimentaire, avec des effets imprévisibles sur les équilibres d'un écosystème déjà compromis par les activités humaines.

La réduction progressive du nombre de filtres solaires organiques considérés sûrs pour l'utilisation humaine et pour l'environnement complique de plus en plus la formulation de produits cosmétiques solaires, notamment des produits solaires à haut facteur de protection, c'est-à-dire avec un SPF supérieur à 30 voire à 50. En effet, traditionnellement, pour obtenir un tel SPF, les formulations cosmétiques pouvaient associer au moins 5 filtres organiques et/ou écrans minéraux.

Parmi les filtres organiques autorisés en Europe qui sont considérés parfaitement sûrs pour l'utilisation chez l'homme, on retrouve des filtres solaires dérivés de triazine, notamment de 1,3,5-triazine, 1,2,4-triazine et 1,2,4-triazine. Ce type de dérivés de triazine correspond, par exemple, aux composés répondant aux désignations INCI suivantes : ethylhexyl triazone, tris-biphenyl triazine, diethylhexyl butamido triazone, phenylene bis-diphenyltriazine, ethylhexyl bis-isopentylbenzoxazolylphenyl melamine ou encore bis-ethylhexyloxyphenol methoxyphenyl triazine.

Cette catégorie de filtres solaires organiques possède des propriétés photoprotectrices très intéressantes car les dérivés de triazine couvrent de larges portions du spectre UV-A et UV-B. En outre, ils sont extrêmement stables notamment parce qu'ils peuvent se photostabiliser réciproquement et participent également à photostabiliser d'autres filtres UV.

Cependant, les filtres dérivés de triazine peuvent se révéler difficile à solubiliser, notamment dans des compositions qui en contiennent de hautes teneurs, c'est-à-dire plus de 8% en poids de la composition. Typiquement, pour la solubilisation des filtres organiques dérivés de triazine on utilise des solubilisants organiques, éventuellement supplémentés avec des filtres organiques à base de salicylate ou encore avec le filtre solaire correspondant à la désignation INCI octocrylene.

A titre d'exemple, les documents XP055656407, DE 10 2007 017436, DE 2007 024347, EP 2 561 853, WO 2017/198806, US 2014/093459 ou encore EP 2 269 567 décrivent des compositions solaires comprenant une teneur en filtres dérivés de triazine strictement inférieure à 10% en poids de la composition. Ces filtres sont combinés à des solubilisants et co-solubilisants, comme l'octocrylène. De telles compositions présentent une faible efficacité de protection solaire et ne garantissent pas une stabilité des filtres dérivés de triazine en formulation.

Ces solutions techniques sont décrites, par exemple, dans les documents EP 1 180 360 et EP 1 034 778. Plus particulièrement, le document EP 1 034 778 décrit la solubilisation de la bis-ethylhexyloxyphenol methoxyphenyl triazine (INCI) par l'ajout dans la formulation d'octocrylene (INCI). Cependant, comme mentionné précédemment, l'octocrylene (INNCI) est soupçonné d'être un perturbateur endocrinien, ce qui limite l'utilité pratique de cet enseignement technique.

Le document EP 1 180 360 décrit la solubilisation du même filtre dérivé de triazine dans le dicaprylyl carbonate (INCI) ou CETIOL^{™} CC. Cependant, le dicaprylyl carbonate est un émollient qui ne solubilise pas efficacement les filtres dérivés de triazine lorsqu'ils sont présents dans une teneur élevée et/ou lorsque plusieurs types de filtres dérivés de triazine sont utilisés. En conséquence, la protection solaire conférée par les compositions décrites dans ce document ne bénéficient pas de la filtration différentielle et complémentaire offerte par une teneur élevée et/ou plusieurs types de filtres dérivés de triazine.

En outre, comme le mentionne le document EP 1 180 360, ces dérivés de triazine, même solubilisés selon les techniques mentionnés précédemment, ne sont pas stables et recristallisent souvent en solution.

Il subsiste donc un besoin évident de mettre au point des compositions sûres pour l'homme, qui ne soient pas écotoxiques, qui soient efficaces pour filtrer les UV avec un SPF élevé et qui permettent de solubiliser efficacement et durablement les filtres solaires organiques dérivés de triazines sans recristallisation de ces filtres solaires.

### EXPOSE DE L'INVENTION

Le Demandeur a constaté qu'une composition comprenant au moins trois solubilisants choisis spécifiquement et au moins un filtre solaire dérivé de triazine permet de répondre aux besoins mentionnés précédemment.

Au sens de l'invention, par « solubilisant », on désigne un composé qui permet de solubiliser, disperser et/ou dissoudre au moins un filtre solaire dérivé de triazine de manière efficace et durable, c'est-à-dire en le stabilisant sous sa forme solubilisée et en empêchant ou réduisant sa recristallisation ou précipitation en formulation pendant toute la durée d'utilisation du produit.

Au sens de l'invention, par « filtre solaire dérivé de triazine », on désigne une molécule capable de filtrer les UV-A et/ou les UV-B et comprenant au moins un hétérocycle aromatique contenant trois atomes d'azote. Le filtre solaire dérivé de triazine peut donc correspondre à un filtre solaire dérivé de 1,3,5-triazine, une 1,2,4-triazine ou une 1,2,3-triazine.

Le présent texte décrit une composition comprenant :
- au moins trois solubilisants choisis dans le groupe comprenant les composés correspondant aux désignations INCI suivantes : caprylyl caprylate/caprate, dibutyl adipate, dicaprylyl carbonate, diisopropyl sebacate, dicaprylyl ether, coco-caprylate, C12-15 alkyl benzoate, propylheptyl caprylate, butylène glycol dicaprylate/dicaprate, dipropylene glycol dibenzoate, neopentyl glycol diheptanoate, triheptanoin, C12-13 alkyl lactate, ethylhexyl benzoate, C12-C15 alkyl lactate, C12-13 alkyl tartrate, tridecyl salicylate, lauryl lactate, diethyl adipate, diisobutyl adipate, diisopropyl adipate, diethylhexyl adipate, diethylhexyl succinate, propanediol dicaprylate, propylene glycol dicaprylate/dicaprate, isopropyl lauroyl sarcosinate, propylene glycol dibenzoate, hydroxyl dimethoxybenzyl malonate, phenoxyethyl caprylate, isodecyl salicylate, dimethyl capramide et phenethyl benzoate ; et
- au moins un filtre solaire dérivé de triazine.

Avantageusement, la composition selon l'invention est une composition cosmétique et/ou dermatologique.

La composition selon l'invention permet de solubiliser une haute teneur en filtres solaires dérivés de triazine, c'est-à-dire une quantité supérieure à 10% en poids total de la composition, avantageusement supérieure à 15%

Des solubilisants aptes à être mis en oeuvre dans une composition selon l'invention sont disponibles sur le marché auprès de plusieurs fournisseurs. A titre d'exemple, les matières premières suivantes peuvent être mises en oeuvre dans la composition selon l'invention :
- plusieurs matières premières de la gamme CETIOL^{™} commercialisées par la société BASF, notamment le CETIOL^{™} RLF, CETIOL^{™} B, CETIOL^{™} CC, CETIOL^{™} O, CETIOL^{™} C5, CETIOL^{™} AB, CETIOL^{™} SENSOFT correspondant respectivement aux désignations INCI caprylyl caprylate/caprate, dibutyl adipate, dicaprylyl carbonate, dicaprylyl ether, coco-caprylate, C12-15 alkyl benzoate, propylheptyl caprylate ;
- plusieurs matières premières de la gamme DUB^{™} commercialisées par la société STEARINERIE DUBOIS, notamment le DUB^{™} DIS, DUB^{™} DEA, DUB^{™} DIBA, DUB^{™} ZENOAT correspondant respectivement aux désignations INCI diisopropyl sebacate, diethyl adipate, diisobutyl adipate et propanediol dicaprylate ;
- le MIGLYOL^{™} 8810 et T-C7 commercialisés par la société IOI OLEO GmbH correspondant respectivement aux désignations INCI butylène glycol dicaprylate/dicaprate et triheptanoin ;
- le LEXSOLV^{™} A commercialisé par la société INOLEX correspondant à la désignation INCI dipropylene glycol dibenzoate (and) neopentyl glycol diheptanoate ;
- plusieurs matières premières de la gamme COSMACOL^{™} commercialisées par la société SASOL ITALY, notamment COSMACOL^{™} ELI, COSMACOL^{™} ETI, COSMACOL^{™} ESI et COSMACOL^{™} LL, correspondant respectivement aux désignations INCI C12-13 alkyl lactate, C12-13 alkyl tartrate, tridecyl salicylate et lauryl lactate ;
- le CERAPHYL^{™} 41 ester commercialisé par la société ASHLAND et correspondant à la désignation INCI C12-C15 alkyl lactate ;
- le FINSOLV^{™} EB et le FINSOLV ^{™} PG 22 commercialisés par la société INNOSPEC et correspondant respectivement aux désignations INCI ethylhexyl benzoate et dipropylene glycol dibenzoate ;
- plusieurs matières premières de la gamme CRODAMOL^{™}, notamment CRODAMOL^{™} DA, CRODAMOL^{™} DOA, CRODAMOL^{™} OSU et CRODAMOL^{™} PC commercialisées par la société CRODA et correspondant respectivement aux désignations INCI diisopropyl adipate, diethylhexyl adipate, diethylhexyl succinate et propylene glycol dicaprylate/dicaprate ;
- le ELDEW^{™} SL-205 commercialisé par la société AJINOMOTO et correspondant à la désignation INCI isopropyl lauroyl sarcosinate ;
- le LEXFEEL^{™} SHINE commercialisé par la société INOLEX et correspondant à la désignation INCI propylene glycol dibenzoate ;
- le TEGOSOFT^{™} XC commercialisé par la société EVONIK et correspondant à la désignation INCI phenoxyethyl caprylate ;
- le RONACARE^{™} AP commercialisé par la société MERCK KGAA et correspondant à la désignation INCI hydroxyl dimethoxybenzyl malonate ;
- le DERMOL^{™} IDSA commercialisé par la société ALZO INTL et correspondant à la désignation INCI isodecyl salicylate ;
- le SPECTRASOLV^{™} DMDA commercialisé par la société HALLSTAR et correspondant à la désignation INCI dimethyl capramide ;
- le X-TEND^{™} 226 commercialisé par la société ASHLAND et correspondant à la désignation INCI phenethyl benzoate.

Selon l'invention, la composition comprend au moins quatre solubilisants.

Selon l'invention, la composition comprend au moins les solubilisants correspondant aux désignations INCI suivantes : dibutyl adipate, dicaprylyl carbonate, diisopropyl sebacate et propylheptyl caprylate.

Selon un mode de réalisation particulier de l'invention, le moins un filtre solaire dérivé de triazine est choisi dans le groupe comprenant les filtres solaires dérivés de 1,3,5-triazines, de 1,2,4-triazines et de 1,2,3-triazines, avantageusement les filtres dérivés de 1,3,5-triazines.

Avantageusement, le moins un filtre solaire dérivé de triazine est choisi dans le groupe comprenant les composés correspondant aux désignations INCI suivantes : bis ethylhexyloxyphenol methoxyphenyl triazine, diethylhexyl butamido triazone, tris-biphenyl triazine, ethylhexyl triazone, phenylene bis-diphenyltriazine et ethylhexyl bis-isopentylbenzoxazolylphenyl melamine.

Des filtres solaires dérivés de triazine aptes à être mis en oeuvre dans une composition selon l'invention sont disponibles sur le marché auprès de plusieurs fournisseurs. A titre d'exemple, les matières premières suivantes peuvent être mises en oeuvre dans la composition selon l'invention :
- le TINOSORB^{®} S/TINOSORB^{®} AQUA commercialisé par la société BASF et correspondant à la désignation INCI bis ethylhexyloxyphenol methoxyphenyl triazine (numéro CAS : 187393-00-6) ;
- l' UVASORB^{®} HEB commercialisé par la société SIGMA 3V et correspondant à la désignation INCI diethylhexyl butamido triazone (numéro CAS : 154702-15-5) ;
- le TINOSORB^{®} A2B commercialisé par la société BASF et correspondant à la désignation INCI tris-biphenyl triazine (numéro CAS : 31274-51-8) ;
- l'UVINUL^{®} T150 commercialisé par la société BASF et correspondant à la désignation INCI ethylhexyl triazone (numéro CAS : 88122-99-0) ;
- le TRIASORB^{®} commercialisé par la société PLANTES & INDUSTRIE et correspondant à la désignation INCI phenylene bis-diphenyltriazine (numéro CAS : 55514-22-2) ;
- l'UVASORB^{®} K2A commercialisé par la société SIGMA 3V et correspondant à la désignation INCI ethylhexyl bis-isopentylbenzoxazolylphenyl melamine (numéro CAS : 288254-16-0) ;
- les filtres dérivés de 1,3,5-triazine notamment le 4,4'-[[6-[[4-[[(1,1-dimethylethyl)amino]carbonyl]phenyl]amino]-1,3,5-triazine-2,4-diyl]diimino]bis-, bis(2-ethylhexyl)benzoate (numéro : CAS 2174063-28-4), le 4-[[4-[[4-[[(1,1-dimethylethyl)amino]carbonyl]phenyl]amino]-6-[[4-[[[4-[[(2-ethylhexyl)oxy]carbonyl]phenyl]amino]carbonyl]phenyl]amino]-1,3,5-triazin-2-yl]amino]-,2-ethylhexyl benzoate (numéro CAS : 2174063-29-5) et le 4,4'-[[6-[[4-[[[4-[[(2-ethylhexyl)oxy]carbonyl]phenyl]amino]carbonyl]phenyl]amino]-1,3,5-triazine-2,4-diyl]diimino]bis-, 1,1'-bis(2-ethylhexyl) benzoate (numéro CAS : 2174063-30-8), qui sont décrits dans le document EP 3 275 872 ;
- la 2,4,6-tris(4'-amino benzalmalonate de diéthyle)-s-triazine ; la 2,4,6-tris(4'-amino benzalmalonate de diisopropyle)-1,3,5-triazine ; la 2,4,6-tris(4'-amino benzalmalonate de diméthyle)-1,3,5,triazine ou encore la 2,4,6-tris(a-cyano-4-aminocinnamate d'éthyle)-1,3,5-triazine. Ces composés sont décrits dans le document EP 0 507 692.

Selon un mode de réalisation particulier, la composition selon l'invention comprend :
- au moins trois solubilisants choisis dans le groupe comprenant les composés correspondant aux désignations INCI suivantes : caprylyl caprylate/caprate, dibutyl adipate, dicaprylyl carbonate, diisopropyl sebacate, dicaprylyl ether, coco-caprylate, C12-15 alkyl benzoate, propylheptyl caprylate, butylène glycol dicaprylate/dicaprate, dipropylene glycol dibenzoate, neopentyl glycol diheptanoate, triheptanoin, C12-13 alkyl lactate, ethylhexyl benzoate, C12-C15 alkyl lactate, C12-13 alkyl tartrate, tridecyl salicylate, lauryl lactate, diethyl adipate, diisobutyl adipate, diisopropyl adipate, diethylhexyl adipate, diethylhexyl succinate, propanediol dicaprylate, propylene glycol dicaprylate/dicaprate, isopropyl lauroyl sarcosinate, propylene glycol dibenzoate, hydroxyl dimethoxybenzyl malonate, phenoxyethyl caprylate, isodecyl salicylate, dimethyl capramide et phenethyl benzoate ; et
- deux filtres solaires dérivés de triazine différents choisis dans le groupe comprenant les 1,2,4-triazines, les 1,3,5-triazines et les 1,2,3-triazines, avantageusement choisis dans le groupe comprenant les composés correspondant aux désignations INCI suivantes : bis ethylhexyloxyphenol methoxyphenyl triazine, diethylhexyl butamido triazone, tris-biphenyl triazine, ethylhexyl triazone, phenylene bis-diphenyltriazine et ethylhexyl bis-isopentylbenzoxazolylphenyl melamine.

Selon un mode de réalisation particulier, la composition selon l'invention comprend :
- au moins trois solubilisants choisis dans le groupe comprenant les composés correspondant aux désignations INCI suivantes : caprylyl caprylate/caprate, dibutyl adipate, dicaprylyl carbonate, diisopropyl sebacate, dicaprylyl ether, coco-caprylate, C12-15 alkyl benzoate, propylheptyl caprylate, butylène glycol dicaprylate/dicaprate, dipropylene glycol dibenzoate, neopentyl glycol diheptanoate, triheptanoin, C12-13 alkyl lactate, ethylhexyl benzoate, C12-C15 alkyl lactate, C12-13 alkyl tartrate, tridecyl salicylate, lauryl lactate, diethyl adipate, diisobutyl adipate, diisopropyl adipate, diethylhexyl adipate, diethylhexyl succinate, propanediol dicaprylate, propylene glycol dicaprylate/dicaprate, isopropyl lauroyl sarcosinate, propylene glycol dibenzoate, hydroxyl dimethoxybenzyl malonate, phenoxyethyl caprylate, isodecyl salicylate, dimethyl capramide et phenethyl benzoate ; et
- trois filtres solaires dérivés de triazine différents choisis dans le groupe comprenant les 1,2,4-triazines, les 1,3,5-triazines et les 1,2,3-triazines, avantageusement choisis dans le groupe comprenant les composés correspondant aux désignations INCI suivantes : bis ethylhexyloxyphenol methoxyphenyl triazine, diethylhexyl butamido triazone, tris-biphenyl triazine, ethylhexyl triazone, phenylene bis-diphenyltriazine et ethylhexyl bis-isopentylbenzoxazolylphenyl melamine.

Selon un mode de réalisation particulier, la composition selon l'invention comprend :
- au moins quatre solubilisants choisis dans le groupe comprenant les composés correspondant aux désignations INCI suivantes : caprylyl caprylate/caprate, dibutyl adipate, dicaprylyl carbonate, diisopropyl sebacate, dicaprylyl ether, coco-caprylate, C12-15 alkyl benzoate, propylheptyl caprylate, butylène glycol dicaprylate/dicaprate, dipropylene glycol dibenzoate, neopentyl glycol diheptanoate, triheptanoin, C12-13 alkyl lactate, ethylhexyl benzoate, C12-C15 alkyl lactate, C12-13 alkyl tartrate, tridecyl salicylate, lauryl lactate, diethyl adipate, diisobutyl adipate, diisopropyl adipate, diethylhexyl adipate, diethylhexyl succinate, propanediol dicaprylate, propylene glycol dicaprylate/dicaprate, isopropyl lauroyl sarcosinate, propylene glycol dibenzoate, hydroxyl dimethoxybenzyl malonate, phenoxyethyl caprylate, isodecyl salicylate, dimethyl capramide et phenethyl benzoate ; et
- deux filtres solaires dérivés de triazine différents choisis dans le groupe comprenant les composés correspondant aux désignations INCI suivantes : bis ethylhexyloxyphenol methoxyphenyl triazine, diethylhexyl butamido triazone et ethylhexyl triazone.

Selon un autre mode de réalisation, la composition selon l'invention comprend :
- au moins quatre solubilisants choisis dans le groupe comprenant les composés correspondant aux désignations INCI suivantes : caprylyl caprylate/caprate, dibutyl adipate, dicaprylyl carbonate, diisopropyl sebacate, dicaprylyl ether, coco-caprylate, C12-15 alkyl benzoate, propylheptyl caprylate, butylène glycol dicaprylate/dicaprate, dipropylene glycol dibenzoate, neopentyl glycol diheptanoate, triheptanoin, C12-13 alkyl lactate, ethylhexyl benzoate, C12-C15 alkyl lactate, C12-13 alkyl tartrate, tridecyl salicylate, lauryl lactate, diethyl adipate, diisobutyl adipate, diisopropyl adipate, diethylhexyl adipate, diethylhexyl succinate, propanediol dicaprylate, propylene glycol dicaprylate/dicaprate, isopropyl lauroyl sarcosinate, propylene glycol dibenzoate, hydroxyl dimethoxybenzyl malonate, phenoxyethyl caprylate, isodecyl salicylate, dimethyl capramide et phenethyl benzoate ; et
- trois filtres solaires dérivés de triazine différents choisis dans le groupe comprenant les composés correspondant aux désignations INCI suivantes : bis ethylhexyloxyphenol methoxyphenyl triazine, diethylhexyl butamido triazone et ethylhexyl triazone.

Selon un mode de réalisation particulier, la composition selon l'invention telle que décrite précédemment comprend au moins les filtres solaires dérivés de triazine correspondant aux désignations INCI suivantes : bis ethylhexyloxyphenol methoxyphenyl triazine, diethylhexyl butamido triazone et ethylhexyl triazone.

Selon un mode de réalisation particulier, le au moins un filtre solaire dérivé de triazine représente une quantité supérieure à 10% en poids total de la composition, avantageusement supérieure à 15%.

Selon un mode de réalisation particulier, les solubilisants représentent entre 5% et 80% en poids total de la composition, avantageusement entre 10% et 70%, de préférence entre 15% et 60%.

Selon un autre mode de réalisation, outre le au moins un filtre solaire dérivé de triazine, la composition selon l'invention comprend au moins un filtre UV-A et/ou UV-B, organique et/ou minéral, qui peut se présenter en phase aqueuse (hydrophile) et/ou huileuse (lipophile).

Selon un mode de réalisation particulier, la composition selon l'invention comprend au moins un filtre UV-A, susceptible d'assurer une filtration complète de la partie nuisible du spectre solaire.

Selon un mode de réalisation particulier, la composition selon l'invention comprend au moins un filtre solaire UV-A choisi dans le groupe comprenant les composés correspondant aux désignations INCI suivantes : butyl methoxydibenzoylmethane, diethylamino hydroxybenzoyl hexyl benzoate, bis-(diethylaminohydroxybenzoyl benzoyl) piperazine, disodium phenyl dibenzimidazole tetrasulfonate et leurs mélanges.

A titre d'exemple, les filtres UV-A selon l'invention correspondent à la matière première Parsol 1789^{®} (INCI : butyl methoxydibenzoylmethane) commercialisée par la société DSM ; UVINUL^{®} A+ (INCI : diethylamino hydroxybenzoyl hexyl benzoate) commercialisée par la société BASF ou encore la matière première C1332^{®} (INCI : bis-(diethylaminohydroxybenzoyl benzoyl) piperazine ; numéro CAS 919803-06-8) commercialisée par la société BASF.

Avantageusement, la composition selon l'invention comprend un filtre UV-A correspondant à la désignation INCI disodium phenyl dibenzimidazole tetrasulfonate, notamment disponible sous la dénomination Neo Heliopan^{®} AP et commercialisé par la société SYMRISE.

Selon un mode de réalisation particulier, la composition selon l'invention comprend des écrans minéraux (ou filtres minéraux inorganiques), qui correspondent à des oxydes métalliques et/ou d'autres composés difficilement solubles ou insolubles dans l'eau, en particulier les oxydes de titane (TiO₂), de zinc (ZnO), de fer (Fe₂O₃), de zirconium (ZrO₂), de silicium (SiO₂), de manganèse (par exemple MnO), d'aluminium (Al₂O₃), ou de cérium (Ce₂O₃).

Avantageusement, les filtres minéraux inorganiques peuvent être utilisés sous forme de prédispersion huileuse ou aqueuse disponible sur le marché. Ces prédispersions peuvent être additionnées avantageusement d'auxiliaires de dispersion et/ou de médiateurs de solubilisation.

Les filtres minéraux inorganiques peuvent également être traités en surface ou encapsulés, afin de leur conférer un caractère hydrophile, amphiphile ou hydrophobe. Ce traitement de surface peut consister en ce que les filtres minéraux soient dotés d'une mince pellicule inorganique et/ou organique hydrophile et/ou hydrophobe.

Selon un mode de réalisation particulier, la composition selon l'invention comprend au moins un écran minéral choisi dans le groupe comprenant les composés correspondant aux désignations INCI suivantes : zinc oxide, titanium dioxide et leurs mélanges.

A titre d'exemple, le zinc oxide correspond à la matière première Z-COTE^{®} LSA et le titanium dioxide à la matière première T-Lite^{®}, commercialisées par la société BASF.

Les listes des filtres UV cités pouvant être mis en oeuvre au sens de la présente invention sont bien entendu donnés à titre indicatif et non limitatif.

De manière avantageuse, le au moins un filtre solaire et/ou écran minéral, à l'exception du ou des filtre(s) solaire(s) dérivé(s) de triazine, représente entre 0,1% et 30% en poids total de la composition selon l'invention, avantageusement entre 0,5% et 20%, encore plus avantageusement entre 1% et 15%.

Selon un mode de réalisation particulier, la composition selon l'invention est exempte des filtres solaires correspondant aux désignations INCI suivantes : 4-methylbenzylidene camphor, benzophenone-2, benzophenone-3, ethylhexyl methoxycinnamate et octocrylene.

Selon un mode de réalisation particulier, la composition selon l'invention présente un SPF égal ou supérieur à 30, avantageusement égal ou supérieur à 50, de préférence égal ou supérieur à 70, voire égal ou supérieur à 100.

Selon un mode de réalisation préféré, la composition selon l'invention comporte un ratio de protection UV-A/UV-B égal ou supérieur à 1/3.

Avantageusement, la composition selon l'invention présente une longueur d'onde critique (λc) supérieure à 370 nm. Cette valeur, qui est déterminée par des méthodes *in vitro* connues de l'homme du métier, correspond à la longueur d'onde pour laquelle l'intégrale de la courbe du spectre d'absorption commençant à 290 nm atteint 90 % de l'intégrale entre 290 et 400 nm.

La composition selon l'invention, peut en outre comprendre un « booster » de SPF, c'est-à-dire un agent amplificateur du facteur de protection solaire, et/ou un photostabilisant, c'est à dire un ingrédient qui permet d'augmenter le SPF ou de photostabiliser les filtres, un tel ingrédient n'étant pas considéré lui-même comme un filtre solaire. On peut par exemple citer :
- le butyloctyl salicylate (INCI), photostabilisant représentant avantageusement entre 0,01% et 10% en poids total de la composition, encore plus avantageusement ente 0,1% et 2%. Cette matière première est, par exemple, commercialisée par la société HALLSTAR sous le nom de Hallbrite^{®} BHB ;
- le benzotriazolyl dodecyl p-cresol (INCI), photostabilisant représentant avantageusement entre 0,01% et 10% en poids total de la composition, encore plus avantageusement entre 0,1% et 2%. Cette matière première est, par exemple, commercialisée par la société BASF sous le nom de TINOGARD^{®} TL ;
- le pongamol (INCI), molécule végétale absorbant dans les UV-A, représentant avantageusement entre 0,5% et 2% en poids total de la composition, encore plus avantageusement de l'ordre de 1%. A titre d'exemple, la matière première Pongamia Extract commercialisée par la société GIVAUDAN peut être utilisée dans le cadre la présente invention ;
- l'ethylhexyl methoxycrylene (INCI), photostabilisant, solubilisant et « booster » de SPF représentant avantageusement entre 1% et 5% en poids total de la composition. La matière première SolaStay^{®} S1 commercialisée par la société HALLSTAR peut être utilisée dans le cadre de la présente invention ;
- un copolymère de styrène acrylate (INCI : styrene/acrylate copolymer), représentant de préférence entre 1% et 10% en poids total de la composition selon l'invention. Les matières premières SunSpheres^{®} H53 et SunSpheres^{®} PGL Polymer, commercialisées par la société DOW CHEMICALS, peuvent être utilisées dans le cadre de la présente invention ;
- le diethylhexyl syringylidene malonate (INCI), représentant avantageusement entre 1% et 10% en poids total de la composition. La matière première OXYNET^{®} ST, commercialisée par la société MERCK, peut être utilisée dans le cadre de la présente invention ;
- un polyester hydrodispersible, correspondant aux désignations INCI polyester-5 (and) Sodium silicoaluminate, représentant avantageusement entre 1% et 10% en poids total de la composition, notamment l'EASTMANN AQTM38S Polymer commercialisé par la société SAFIC-ALCAN ;
- un copolymère d'acrylate ayant une température de transition vitreuse de -5°C à -15°C telle que mesurée par calorimétrie différentielle à balayage, ledit copolymère représentant avantageusement entre 1% et 10% en poids total de la composition. Par exemple, un polymère correspondant à la désignation INCI Acrylate copolymer, tel que la matière première EPITEX 66, commercialisée par la société DOW CHEMICALS, peut être utilisée dans le cadre de la présente invention.

Selon un autre mode de réalisation, la composition selon l'invention peut comprendre, en outre, un extrait de la bactérie *Arthrobacter agilis,* notamment un extrait riche en caroténoïdes, tel que décrit dans le document WO 2014/167247. Avantageusement, la composition selon l'invention comprend de 0,00001% à 0,1% en poids total de la composition, encore plus avantageusement de 0,0001% à 0,001 % d'un tel extrait sec.

Dans un mode de réalisation privilégié, la composition selon l'invention comprend, en outre, d'autres composants pouvant contribuer à la protection interne par une action qui peut consister en une protection de l'ADN, une diminution de l'immunosuppression induite par les radiations UV, une action antiradicalaire ou un effet combiné de ces actions.

L'action protectrice d'une préparation selon l'invention contre le stress oxydatif ou à l'encontre de l'effet des radicaux libres peut être encore améliorée si celle-ci comprend, en outre, un ou plusieurs antioxydants, aisément sélectionnés par l'homme du métier par exemple dans la liste suivante : le totarol, le magnolol, l'honokiol, les acides aminés et leurs dérivés, des peptides (D et/ou L-carnosine) et leurs dérivés (par exemple l'ansérine, l'hypotaurine, la taurine), les caroténoïdes, les carotènes (α-carotène, β-carotène, lycopène) et leurs dérivés, l'acide chlorogénique et ses dérivés, l'acide lipoïque et ses dérivés (acide dihydrolipoïque), l'aurothioglucose, le propylthiouracile et autres thiols (thiorédoxine, glutathion, cystéine, cystine, cystamine et leurs esters glycosyle, N-acétyle, méthyle, éthyle, propyle, amyle, butyle et lauryle, palmitoyle, oléyle, γ-linoléique, cholestéryle et glycéryle) ainsi que des sels de ceux-ci, le thiodipropionate de dilauryle, le thiodipropionate de distéaryle, l'acide thiodipropionique et ses dérivés, les composés sulfoximine (sulfoximine de buthionine, l'homocystéine sulfoximine, les buthionine sulfones, penta-, hexa- et heptathionine sulfoximine), des agents chélatants (tels que les acides α-hydroxygras, l'acide palmitique, l'acide phytique, la lactoferrine), les α-hydroxyacides (tels que l'acide citrique, lactique, ou malique), l'acide humique, l'acide biliaire, les extraits de bile, la bilirubine, la biliverdine, le tétraméthylène phosphonate pentasodique d'éthylènediamine et ses dérivés, les acides gras insaturés et leurs dérivés, la vitamine A et ses dérivés (palmitate de vitamine A), le benzoate de coniféryle de la résine de benjoin, l'acide rutinique et ses dérivés, l'α-glycosyl rutine, l'acide férulique et ses dérivés, le furfurylideneglucitol, la carnosine, le butylhydroxytoluène, le butylhydroxyanisole, l'acide nordihydroguaiarétique, trihydroxybutyrophenone, la quercétine, l'acide urique et ses dérivés, le mannose et les dérivés de ceux-ci, le zinc et ses dérivés (ZnO, ZnSO4), le sélénium et ses dérivés (sélénométhionine), les stilbènes et leurs dérivés (l'oxyde de stilbène, l'oxyde trans-stilbène).

Dans un mode de réalisation particulier, la composition selon l'invention comprend, en outre, de l'acide glycyrrhétinique, un dérivé ou un sel de cet acide, utilisé comme apaisant (agent anti-inflammatoire) et représentant entre 0,01% et 2 % en poids total de la composition, de préférence entre 0,1% et 1%.

Selon une autre caractéristique privilégiée de l'invention, la composition cosmétique et/ou dermatologique comprend au moins un, voire tous les constituants suivants exerçant une activité biologique *in vivo* sur les cellules de la peau, des lèvres, des cheveux et/ou des muqueuses soumises à un rayonnement UV-A et/ou UV-B, respectivement :
- un agent antiradicalaire préservant les structures cellulaires, tel que par exemple la vitamine E et/ou ses dérivés liposolubles ou hydrosolubles, en particulier le tocotriénol et/ou le tocophérol, représentant avantageusement entre 0,001% et 10% en poids total de la composition, encore plus avantageusement entre 0,02% et 2%, de préférence de l'ordre de 0,04%;
- un agent limitant l'immunosuppression, tel que par exemple la vitamine PP, représentant avantageusement entre 0,001% et 1% en poids total de la composition, encore plus avantageusement de 0,01% à 0,3%;
- un agent protecteur de la protéine p53, tel que par exemple l'épigallocatéchine gallate (EGCG), représentant avantageusement entre 0,001% et 0,1% en poids total de la composition, encore plus avantageusement de 0,005% à 0,05%.

La composition selon l'invention peut également comprendre, en outre, des extraits peptidiques de soja et/ou de blé, tels que ceux décrits dans le document EP 2 059 230.

En pratique, les extraits peptidiques proviennent de graines de soja et de blé sont issus d'une hydrolyse enzymatique desdites graines par l'intermédiaire de peptidases qui permettent de récupérer des peptides d'une taille moyenne de 700 Daltons. Préférentiellement, l'extrait peptidique de soja est l'extrait identifié sous le numéro CAS 68607-88-5 de même que l'extrait peptidique de blé est l'extrait identifié sous le numéro CAS 70084-87-6. Les extraits de blé et soja peuvent correspondre aux désignations INCI Hydrolyzed wheat protein et Hydrolyzed soy protein, respectivement.

Dans un mode de réalisation particulier, les extraits peptidiques de soja et/ou de blé sont utilisés ensemble, par exemple dans un rapport pondéral respectivement compris entre 80/20 et 20/80, avantageusement compris entre 70/30 et 30/70, de préférence égal à 60/40.

Dans un mode de réalisation avantageux, les extraits peptidiques de soja et/ou de blé sont exempts de tripeptides synthétiques GHK (glycyl-histidyl-lysine ; INCI : Tripeptide-1). En pratique, les extraits peptidiques de soja et/ou blé représentant de 0,01 à 20 % en poids total de la composition, avantageusement de 0,1% à 10 %, encore plus avantageusement de 0,2% à 0,7%.

Dans un mode de réalisation alternatif, la composition selon l'invention comprend, conformément aux enseignements du document FR 2 865 398, l'association d'au moins un acide aminé choisi dans le groupe constitué par l'ectoïne, la créatine, l'ergothionéine et/ou la carnosine, ou leurs sels physiologiquement acceptables, et du mannitol ou un dérivé du mannitol.

De préférence, la composition selon l'invention comprend dans un milieu physiologiquement acceptable l'acide aminé ou l'un de ses sels, seul ou en mélange dans des proportions comprises entre 0,001% et 10 % en poids total de la composition, et de préférence entre 0,01% et 5 %.

La composition selon la présente invention comprend, de préférence, dans un milieu physiologiquement acceptable, du mannitol ou l'un de ses dérivés, dans des proportions comprises entre 0,01% et 30 % en poids total de la composition, avantageusement entre 0,1% et 10 %.

Dans un mode de réalisation privilégié, la composition selon l'invention comprend de l'ectoïne et du mannitol.

Selon un mode de réalisation particulier, la composition selon l'invention comprend un ou plusieurs autres agents bronzants ou autobronzants. Il peut s'agir d'un autobronzant qui réagit avec les acides aminés de la peau selon une réaction de Maillard ou par l'intermédiaire d'une addition de Michael, ou bien un promoteur de la mélanogénèse ou un composé propigmentant qui favorise le bronzage naturel de la peau.

Une telle substance est de préférence présente dans la composition en quantité allant de 0,01% à 20% en poids total de la composition, avantageusement de 0,5% à 15%, encore plus avantageusement de 1% à 8%.

Les substances autobronzantes peuvent être le 1,3-dihydroxyacétone (DHA), le glycérolaldéhyde, l'hydroxyméthylglyoxal, l'γ-dialdéhyde, l'érythrulose, le 6-aldo-D-fructose, la ninhydrine, la 5-hydroxy-1,4-naphtoquinone (juglone), la 2-hydroxy-1,4-naphtoquinone (lawsone), ou leur combinaison.

Les substances propigmentantes peuvent être l'hormone stimulant les mélanocytes (α-MSH), des analogues peptidiques de l'α-MSH, des agonistes du récepteur à l'endothéline-1, des agonistes des récepteurs µ opiacés, des agents stimulateurs d'AMPc, des agents stimulateurs de tyrosinase.

Dans un mode de réalisation particulier, la composition selon l'invention comprend, en outre, en qualité d'autobronzant, une combinaison de dihydroxy méthylchromonyl palmitate et/ou le dimethylmethoxy chromanol ainsi qu'une forme lipophile de la tyrosine.

Cette association de principes actifs permet de stimuler efficacement le bronzage. Le dihydroxy méthylchromonyl palmitate (numéro CAS : 1387636-35-2) correspond, par exemple, à l'ingrédient cosmétique commercialisé par la société MERCK sous le nom de RonaCare^{®} Bronzyl. Le dimethylmethoxy chromanol (numéro20CAS : 83923-51-7) correspond, par exemple, à l'ingrédient cosmétique commercialisé par la société LIPOTEC SA sous le nom de lipochromone-6.

Selon un mode de réalisation particulier, le dihydroxy méthylchromonyl palmitate ou le dimethylmethoxy chromanol est compris dans la composition selon l'invention à hauteur de 0,01% à 10% en poids total de la composition, avantageusement de 0,05% à 10%, encore plus avantageusement de 0,1% à 5%, plus particulièrement de 0,1% à 0,5%.

La forme lipophile de la tyrosine, au sens de l'invention, est un ingrédient à base de tyrosine et présente un caractère lipophile plus prononcé que la tyrosine. La forme lipophile de la tyrosine peut notamment correspondre à l'oléoyl tyrosine (Numéro CAS : 147732-57-8), qui se retrouve, par exemple, dans l'ingrédient cosmétique liquide TYR-OL, commercialisé par la société SEDERMA, et qui comprend environ 50% en poids d'oléoyl tyrosine dans du butylène glycol (environ 30% + environ 20% d'acide oléique), ou bien dans l'ingrédient cosmétique liquide TYR-EXCEL, commercialisé par la société SEDERMA, qui comprend environ 50% en poids d'oléoyl tyrosine, environ 20% en poids d'acide oléique (N° CAS : 112-80-1) et environ 30% en poids d'huile de *Luffa cylindrica* (huile de pépins de courge éponge; N° CAS : 1242417-48-6).

Selon un autre mode de réalisation, la forme lipophile de la tyrosine correspond à une huile végétale dans laquelle a été formulée la tyrosine.

Selon un mode de réalisation particulier, l'huile végétale est de l'huile de tournesol oléique, avantageusement désodorisée. Ainsi, la matière première OLEOACTIF TYROSINE BASE HELIANTHUS ANNUS commercialisée par l'entreprise OLEOS, et correspondant aux désignations INCI *Helianthus annuus* seed oil (and) tyrosine (and) glyceryl stéarate, peut être utilisée dans le cadre de la présente invention.

En pratique, la forme lipophile de la tyrosine, telle que dans les ingrédients cosmétiques à base d'oléoyl-tyrosine (avantageusement à 50% en poids) ou de la tyrosine formulée dans de l'huile végétale, représente entre 0,1% et 10% en poids total de la composition, avantageusement entre 1% et 3%, encore plus avantageusement entre 1% et 1,5%.

La composition selon l'invention peut également comprendre des actifs ayant des propriétés dépigmentantes, comme par exemple :
- de l'azéilate de lysine, ou d'autres dérivés ou sels de l'acide azélaïque ;
- de l'andrographolide, notamment l'extrait *d'Andrographis paniculata* correspondant à la désignation INCI *Andrographis paniculata* leaf extract ;
- de l'acide ascorbique natif (vitamine C) ou ses dérivés, notamment les dérivés correspondant aux INCI Ascorbyl Glucoside, Ethyl ascorbic acid, Ascorbyl methylsilanol pectinate, Sodium ascorbyl phosphate et Ascorbyl tetraisopalmitate, avantageusement l'ascorbyl glucoside ;
- de l'arbutine ou un extrait végétal la contenant, notamment l'extrait de busserole correspondant à la désignation INCI *Arctostaphylos uva-ursi* leaf extract ;
- de la glabridine ou un extrait végétal la contenant, notamment les extraits de réglisse correspondant à la désignation INCI *Glycyrrhiza glabra* root extract, *Glycyrrhiza inflata* root extract, *Glycyrrhiza uralensis* root extract ;
- les peptides biomimétiques correspondant aux désignations INCI hexapeptide 2 et/ou nonapeptide-1 ;
- un extrait aqueux d'une algue dénommée *Palmaria palmata*, notamment l'extrait correspondant à la désignation INCI *Palmaria palmata* extract ;
- le 4-n-butylresorcinol ;
- de la vitamine PP, également appelée niacinamide ou nicotinamide, et ses dérivés;
- ou leurs mélanges.

La composition selon l'invention peut également comprendre des actifs ayant des propriétés cicatrisantes comme par exemple un agent antimicrobien choisi parmi les actifs correspondant aux désignations INCI suivantes : copper sulfate, zinc sulfate, sodium hyaluronate, *Vitis vinifera* (grape) vine extract et leurs mélanges.

La composition selon l'invention peut également comprendre des actifs ayant des propriétés sébocorrectices, kératolytiques, séboregulatrices et/ou une activité antiacnéique, afin de permettre la formulation de produits solaires traitant l'acné.

Par exemple, la composition selon l'invention peut comprendre un agent antimicrobien choisi parmi les actifs correspondant aux désignations INCI propyl gallate, dodecyl gallate, *Ginkgo biloba* leaf extract, bakuchiol, dihydromyricetine, zinc gluconate, salicylic acid et leurs mélanges.

La composition selon l'invention peut donc en outre comprendre au moins un ingrédient choisi dans la liste suivante :
- un agent apte à filtrer la lumière visible, en particulier la lumière bleue ;
- un extrait des algues *Laminaria ochroleuca*, *Blidingia minima* ou *Laminaria saccharina* ;
- un extrait de la plante *Zanthoxylum alatum* ;
- du panthénol ;
- un extrait de bois de cade ;
- un extrait de Boldo ;
- un extrait de Reine des prés ;
- un extrait d'huile de karanja issue du *Pongamia glabra* ;
- des paraffines linéaires ;
- de l'ATP (adénosine-5 tri-phosphate), du Gp4G (diguanosine tétraphosphate) ou de l'Ap4A (diadénosine tétraphosphate),
- un acide aminé choisi dans le groupe constitué de la décarboxycarnosine, la glutamine et leurs sels.

La composition selon l'invention peut également comprendre des adjuvants comme ceux habituellement utilisés dans le domaine de la cosmétique, tels que des conservateurs, des antioxydants, des agents complexants, des solvants, des parfums, des charges, des bactéricides, des électrolytes, des absorbeurs d'odeur, des matières colorantes ou encore des vésicules lipidiques. Le choix de ces adjuvants, ainsi que leurs concentrations, doivent être déterminés de telle sorte qu'ils ne modifient pas les propriétés et les avantages recherchés pour la composition de la présente invention.

La composition de l'invention est pour application topique et plus particulièrement pour application sur la peau, les lèvres, les cheveux et/ou les muqueuses.

Ainsi et dans le cadre de l'invention, une telle composition est notamment destinée à la protection de la peau et/ou des phanères, en particulier les muqueuses, les lèvres et les cheveux, contre le rayonnement UV.

La composition de l'invention peut se présenter sous toutes les formes galéniques normalement utilisées dans les domaines cosmétique et dermatologique, comme par exemple, mais de façon non limitative, sous la forme d'une solution aqueuse éventuellement gélifiée, d'une dispersion du type lotion, d'une émulsion H/E ou inversement E/H, plus ou moins fluide, ou d'une émulsion multiple comme par exemple une émulsion triple (E/H/E ou H/E/H), ou encore sous la forme d'une dispersion vésiculaire de type ionique (liposomes) et/ou non ionique, d'une composition biphasée dépourvue d'émulsionnants et gélifiants dont les phases immiscibles se séparent pendant le stockage, de mousse, de stick, d'huile anhydre, de spray ou de brume.

Dans un mode de réalisation préféré, la composition selon l'invention est une émulsion E/H. Avantageusement, les émulsions E/H selon l'invention comprennent le PEG-30 dipolyhydroxystearate (INCI), ou le polyglyceryl-4 diisostearate/polyhydroxystearate/sebacate en qualité d'émulsionnants. Ces matières premières sont disponibles respectivement auprès de CRODA sous le nom commercial de Cithrol DPHS et auprès d'EVONIK sous nom d'Isolan GPS.

Dans un mode de réalisation alternatif, la composition selon l'invention est une émulsion H/E. Avantageusement, les émulsions H/E selon l'invention comprennent un émulsionnant choisi dans le groupe suivant de composés identifiés par leur désignation INCI : sodium stearoyl glutamate, potassium cetyl phosphate, glyceryl stearate/PEG-100 stéarate et C20-22 alkyl phosphate/C20-C22 alkyl alcohols, tribehenin PEG-20 esters, C14-C22 alcohols/ C12-20 alkyl glucoside, cetearyl alcohol/coco-glucoside e et leurs mélanges. Ces matières premières sont disponibles auprès de plusieurs fournisseurs. A titre d'exemple, les matières premières EMULGIN SG (INCI : sodium stearoyl glutamate ; fournisseur : BASF) ; EMULIUM 22 (INCI : tribehenin PEG-20 esters; fournisseur GATTEFOSSE) ; SENSANOV WR (INCI: C20-22 alkyl phosphate/C20-C22 alkyl alcohols ; fournisseur : SEPPIC) ; MONTANOV L (INCI : C14-C22 alcohols/ C12-20 alkyl glucoside), AMPHISOL K (INCI : potassium cetyl phosphate ; fournisseur : DSM), MONTANOV 82 (INCI : cetearyl alcohol/coco-glucoside ; fournisseur : SEPPIC) peuvent être employées dans les compositions selon l'invention.

Selon un autre aspect, l'invention concerne une composition telle que décrite précédemment pour son utilisation pour protéger la peau et/ou les muqueuses contre le rayonnement solaire UV.

Selon un autre aspect, l'invention concerne l'utilisation non-thérapeutique d'une composition telle que décrite précédemment pour protéger les phanères contre le rayonnement solaire ultraviolet.

Selon un mode de réalisation particulier, la composition selon l'invention est utilisée pour filtrer le rayonnement UV compris entre 280 et 400 nm, avantageusement entre 300 et 380 nm.

Le présent text décrit un procédé de solubilisation et/ou dispersion d'au moins un filtre dérivé de triazine compris dans une composition cosmétique, représentant avantageusement une quantité supérieure à 10% en poids total de la composition, de préférence supérieure à 15%.

La manière dont l'invention peut être réalisée et les avantages qui en découlent ressortiront mieux des exemples de réalisation qui suivent, donnés à titre indicatif et non limitatif.

### EXEMPLES DE RÉALISATION DE L'INVENTION

Les pourcentages indiqués sont donnés en poids de produit par rapport au poids total de la composition dans les tableaux ci-dessous.

### Exemple 1 - Huiles solaires SPF100

Les formulations des compositions selon l'exemple I sont représentées dans le tableau 1.

**[Tableau 1]**

| | **% INCI** | | |
|---|---|---|---|
| **NOM INCI** | **FORMULE 1 (invention)** | **FORMULE 2 (invention)** | **FORMULE 3 (hors invention)** |
| **Filtre dérivé de triazine selon l'invention** | | | |
| Bis-ethylhexyloxyphenol methoxyphenyl triazine | 3 | 6 | 4 |
| Ethylhexyl triazone | 4,5 | 4,5 | 3 |
| Diethylhexyl butamido triazone | 4 | 9 | 7 |

| **Solubilisant selon l'invention** | | | |
|---|---|---|---|
| Dicaprylyl carbonate | 20,947 | 19,564129 | 10,566829 |
| Diisopropyl sebacate | 13 | 13 | 13 |
| Propylheptyl caprylate | 9,065465 | 9,995 | - |
| Dibutyl adipate | 7 | 8 | 8 |

| **Filtre UV** | | | |
|---|---|---|---|
| Titanium dioxide | 15 | - | - |
| Zinc oxide | 8,001 | 8,001 | 8,001 |
| Diethylamino hydroxybenzoyl hexyl benzoate | 8 | 9 | 9 |
| Ethylhexyl salicylate | - | 4,5 | 4,5 |

| **Autre composant** | | | |
|---|---|---|---|
| Caprylic/capric triglycéride | 3,0861 | 3,0861 | 3,0861 |
| Isododecane | - | - | 14,5 |
| Silica | 2,1 | - | 5 |
| Jojoba esters | 1,5 | - | - |
| Tocopherol | 0,007535 | 0,012371 | 0,006171 |
| Undecane | - | 3,24835 | 6,4967 |
| Tridecane | - | 1,75 | 3,5 |
| Polyhydroxystearic acid | 0,7929 | 0,3429 | 0,3429 |
| *Helianthus annuus* (sunflower) seed oil | - | 0,00015 | 0,000300 |

Tous les ingrédients sont mélangés, à l'exception des écrans minéraux, sont mélangés sur plaque chauffante à 75-78°C sous agitation. On commence le refroidissement et lorsque la composition atteint une température de 40-45°C, les écrans minéraux sont ajoutés à l'aide d'une turbine défloculeuse sous agitation (Rayneri environ 400 rpm).

### Exemple II - Huile solaire SPF100

La formulation de la composition (hors invention) selon l'exemple II est représentée dans le tableau 2.

**[Tableau 2]**

| **NOM INCI** | **% INCI** |
|---|---|
| **Filtre solaire dérivé de triazine selon l'invention** | |
| Bis-ethylhexyloxyphenol methoxyphenyl triazine | 3,5 |
| Diethylhexyl butamido triazone | 9 |

| **Solubilisant selon l'invention** | |
|---|---|
| Dibutyl adipate | 10 |
| Dicaprylyl carbonate | 9,997 |
| Diisopropyl sebacate | 4,80 |

| **Filtre UV** | |
|---|---|
| Diethylamino hydroxybenzoyl hexyl benzoate | 7 |
| Ethylhexyl salicylate | 4,5 |

| **Autre composant** | |
|---|---|
| Propanediol | 5 |
| Aqua/water/eau | 40,148 |
| C20-22 alkyl phosphate | 0,66 |
| C20-22 alcohols | 0,54 |
| C14-22 alcohols | 0,4 |
| Methylpropanediol | 2 |
| Capryloyl glycine | 0,4 |
| Sodium citrate | 0,4 |
| Citric acid | 0,11 |
| Microcrystalline cellulose | 0,88 |
| C12-20 alkyl glucoside | 0,1 |
| Ectoin | 0,1 |
| Mannitol | 0,1 |
| Xylitol | 0,1 |
| Sodium hydroxide | 0,09 |
| Rhamnose | 0,05 |
| Cellulose gum | 0,12 |
| Tocopherol | 0,003 |
| Fructooligosaccharides | 0,001 |
| Caprylic/capric triglyceride | 0,00095 |
| *Laminaria ochroleuca* extract | 0,00005 |

On disperse l'eau, la soude et la capryloyl glycine à l'aide d'une turbine défloculeuse. Par la suite, on ajoute la cellulose qui est également dispersée à froid. A ce stade, on ajoute les polyols.

Séparément on prépare un mélange des solubilisants selon l'invention et tous les filtres solaires en chauffant à 78°C. L'émulsion est finalement réalisée à cette température sous agitation défloculeuse (Rayneri, environ 400 rpm) suivie d'un 1 minute d'homogénéisation (équipement Ultra-turrax^{®}) à vitesse 2.

### Exemple III - Emulsion E/H SPF100

La formulation de la composition (invention) selon l'exemple II est représentée dans le tableau 3.

**[Tableau 3]**

| **NOM INCI** | **% INCI** |
|---|---|
| **Filtre solaire dérivé de triazine selon l'invention** | |
| Ethylhexyl triazone | 4,5 |
| Diethylhexyl butamido triazone | 4 |
| Bis-ethylhexyloxyphenol methoxyphenyl triazine | 3 |
| **Solubilisant selon l'invention** | |
| Diisopropyl sebacate | 5 |
| Dibutyl adipate | 5 |
| Dicaprylyl carbonate | 16,8185 |
| Propylheptyl caprylate | 2,9985 |

| **Filtre UV** | |
|---|---|
| Titanium dioxide | 15 |
| Zinc oxide | 8,001 |
| Diethylamino hydroxybenzoyl hexyl benzoate | 8 |

| **Autre composant** | |
|---|---|
| Aqua/water/eau | 18,77 |
| Caprylic/capric triglyceride | 3,0861 |
| Silica | 2,1 |
| Jojoba esters | 1,5 |
| Peg-30 dipolyhydroxystearate | 1 |
| Polyhydroxystearic acid | 0,7929 |
| Sodium citrate | 0,2 |
| Stearalkonium hectorite | 0,1 |
| Xanthan gum | 0,1 |
| Propylene carbonate | 0,03 |
| Tocopherol | 0,0030 |

Le diethylamino hydroxybenzoyl hexyl benzoate (filtre organique UV-A) et le PEG-30 dipolyhydroxystearate (tensioactif), les filtres dérivés de triazine et les solubilisants selon l'invention, à l'exception du propylheptyl caprylate, sont mélangés sur plaque chauffante à 75-78°C sous agitation jusqu'à solubilisation totale.

La solution est ensuite refroidie jusqu' à atteindre une température de 40-45°C, à laquelle les écrans minéraux sont ajoutés sous agitation défloculeuse (Rayneri, environ 400 rpm).

En parallèle, le propylheptyl caprylate et le stearalkonium hectorite sont mélangés sous agitation défloculeuse à température ambiante jusqu'à dispersion totale; à ce stade, on ajoute ces composants dans la phase grasse principale.

On réalise la phase aqueuse avec le reste des ingrédients, à l'exception de la gomme de xanthane, que l'on ajoute une fois le citrate bien solubilisé.

L'émulsion eau-dans-huile est finalement réalisée en ajoutant la phase aqueuse gélifiée dans la phase grasse sous agitation défloculeuse (Rayneri ~ 400 rpm) suivie d'un 1 minute d'homogénéisation (équipement Ultra-turrax^{®}) à vitesse 2.

### Exemple IV - Evaluation du SPF in vitro et de la photostabilité des compositions selon l'invention

### IV. 1 - But de l'étude

L'objectif de l'étude est d'évaluer la photostabilité et la photoprotection conférée par deux compositions, c'est-à-dire, la formule 3 de l'exemple I (3 filtres UV dérivés de triazine représentant 14% en poids de la composition + 3 solubilisants représentant 31,6% en poids de la composition ; hors invention) et la formule de l'exemple III (3 filtres UV dérivés de triazine représentant 11,5% en poids de la composition + 4 solubilisants représentant 29,8% en poids de la composition ; invention).

### IV.2 - Matériel et méthodes

### IV. 2.1 - Mesure du SPF, de la protection UV-A, du ratio de protection et de la longueur d'onde critique

Une première mesure d'absorption UV est effectuée sur une plaque de PMMA (polyméthylméthacrylate) recouverte de 15 µL de glycérine (blanc), afin de s'affranchir de l'absorption du support.

Ensuite, le produit étudié est prélevé et déposé en petits spots égaux sur la plaque de PMMA (5*5cm). La quantité pesée est de 32,5 mg, correspondant à une concentration de 1,3 mg/cm² L'échantillon est étalé uniformément à l'aide d'un doigtier (doigt de gant latex) préalablement saturé avec le produit à étudier.

La plaque est ensuite déposée à l'obscurité à température ambiante pendant au moins 15min. On irradie la plaque séchée dans le suntest CPS+ (Atlas) pendant 15min à 600W/cm², ce qui correspond à 1 DEM (Dose Erythémale Minimale) pour un phototype III.

On introduit la plaque irradiée dans l'enceinte de mesure du spectrophotomètre UV/visible (Lambda 650S Perkin Elmer), pour effectuer les mesures d'absorption UV entre 290 et 400 nm. Notons qu'une mesure correspond en fait à la moyenne de trois mesures (trois points de la plaque de PMMA sont mesurés).

L'évaluation de la protection UV-A est obtenue de la même façon que le SPF mais en mesurant l'absorption entre 320 nm à 400nm. Le ratio représente le rapport entre SPF/absorption UV-A. Selon la réglementation européenne, ce rapport doit être >1/3 afin d'assurer une protection suffisante dans les UV-A. La longueur d'onde critique correspond la longueur d'onde pour laquelle il y a 90% de l'aire sous la courbe d'absorbance obtenue de 290 à 400nm.

### IV. 2.2 - Mesure de la photostabilité

Le produit doit être homogénéisé avant son application.

Le protocole est identique à celui du point IV.2.1.

La plaque est irradiée à l'aide du simulateur solaire à 600W/m², jusqu'à ce que la dose représente 4DEMs pour un phototype III.

On introduit à nouveau la plaque irradiée dans l'enceinte de mesure du spectrophotomètre UV/visible, pour effectuer les mesures d'absorption UV entre 290 et 400nm.

Notons que nous utiliserons au moins 5 plaques et effectuerons au moins 3 mesures par plaque. Par conséquent, le résultat final correspond à la moyenne d'au minimum 15 mesures. Une plaque témoin est placée à l'obscurité dans la salle du sun test, afin d'annuler les effets thermiques et ne prendre en compte que les effets purement photochimiques.

La photostabilité du produit s'exprime par le ratio de la protection résiduelle après irradiation sur la protection avant irradiation (la protection étant la capacité à réduire l'énergie UV par l'effet d'absorption ou de dispersion du produit).

### IV.3 - Résultats et conclusion

Les résultats sont résumés dans le tableau 4 ci-dessous.

**[Tableau 4]**

| | **Formule 3 exemple I (Hors invention)** | **Formule exemple III (Invention)** |
|---|---|---|
| **SPF** | 108 (± 7) | 132 (± 6) |
| **Absorption UV-A** | 48 (± 3) | 57 (± 2) |
| **Ratio (SPF-UV-A)** | 2,10 | 1,05 |
| **Longueur d'onde critique** | 374 | 374 |
| **Photostabilité** | 4 DEM: 100% | 4 DEM : 99% |
| | 8 DEM: 101% | 8 DEM : 96% |

En conclusion, les deux formules confèrent une protection maximale dans les UV-B et UV-A et sont extrêmement photostables.

### Exemple V - Etude clinique pour la détermination du facteur de protection solaire (FPS ou SPF) de la formule 3 de l'exemple 1 (hors invention)

### V.1 - Méthode

L'étude a été réalisée selon la méthodologie normée ISO24444:2010 en utilisant la formule 3 selon l'exemple I (fle 3 ; hors invention). Les abréviations utilisées dans les tableaux des résultats sont détaillées par le texte de la norme. Ce test inclut une formule témoin pour laquelle un SPF de 15 était attendu. Cinq volontaires ont participé au test.

### V.2 - Résultats et conclusion

Les résultats obtenus avec la composition selon la formule 3 de l'exemple 1 (fle 3) sont résumés dans le tableau 5 ci-dessous.

**[Tableau 5]**

| **Volontaire** | **Sexe** | **Age** | **Phototype** | **ITA°** | **MEDu (µj/cm2)** | **MEDp (µj/cm2)** | **SPFi** |
|---|---|---|---|---|---|---|---|
| 1 | F | 38 | III | 28 | 83520 | 9126000 | 109,3 |
| 2 | F | 55 | III | 56 | 58500 | 6084000 | 104,0 |
| 3 | M | 38 | I | 59 | 24930 | 2714400 | 108,9 |
| 4 | F | 29 | II | 47 | 40300 | 4212000 | 104,5 |
| 5 | F | 55 | I | 56 | 22160 | 2505600 | 113,1 |
| Résultats | | | | | SPF moyen | | 107,9 |
| | | | | | Ecart type | | 3,7 |

Les résultats obtenus avec le témoin de norme sont résumés dans le tableau 6 ci-dessous.

**[Tableau 6]**

| **Volontaire** | **Phototype** | **MEDu (µj/cm2)** | **MEDp (µj/cm2)** | **SPFi** |
|---|---|---|---|---|
| 1 | III | 83520 | 1404000 | 16,8 |
| 2 | III | 58500 | 936000 | 16,0 |
| 3 | I | 24930 | 446400 | 17 ,9 |
| 4 | II | 40300 | 626400 | 15,5 |
| 5 | I | 22160 | 374400 | 16,9 |
| Résultats | | SPF moyen | | 16,6 |
| | | Ecart type | | 0,9 |

Les résultats montrent que la composition fle 3 permet d'obtenir un SPF>100 (en moyenne).

### Exemple VI - Essais de solubilisation de hautes teneurs de filtres solaires dérivés de triazine par les solubilisants selon l'invention et leurs combinaisons

### VI.1 - But de l'étude

L'objectif de l'étude est d'évaluer la stabilité des compositions à haute teneur en filtres solaires dérivés de triazine pendant une période de 2 mois à 4°C (température à laquelle les filtres solaires dérivés de triazine ont tendance à recristalliser).

### VI.2 - Matériel et méthodes

Plusieurs prémélanges (prém) filtres + solubilisants ont été préparés. Les prémélanges sont faits avec les filtres solaires dérivés de triazines et solubilisants des formules 1 et 2 de l'exemple I et dans différentes quantités. Le tocophérol présent est amené par l'une des matières premières formulées dans le prémélange.

Le tableau 7 résume les formulations des différents prémélanges comprenant chacun 11,5% en poids de la composition de filtres solaires dérivés de triazine.

**[Tableau 7]**

| | **% INCI** | | | |
|---|---|---|---|---|
| **Nom INCI Mixte** | **Prém 1 (Invention)** | **Prém 2 (Hors invention)** | **Prém 3 (Hors invention)** | **Prém 4 (Hors invention)** |
| **Filtre solaire dérivé de triazine selon l'invention** | | | | |
| Ethylhexyl triazone | 4,5 | 4,5 | 4,5 | 4,5 |
| Diethylhexyl butamido triazone | 4 | 4 | 4 | 4 |
| Bis-ethylhexyloxyphenol methoxyphenyl triazine | 3 | 3 | 3 | 3 |

| **Autre filtre** | | | | |
|---|---|---|---|---|
| Diethylamino hydroxybenzoyl hexyl benzoate | 8 | 8 | 8 | 8 |

| **Solubilisant** | | | | |
|---|---|---|---|---|
| Diisopropyl sebacate | 13 | 13 | 13 | 13 |
| Dicaprylyl carbonate | 9,997 | 9,997 | - | - |
| Propylheptyl caprylate | 9,065465 | - | - | - |
| Dibutyl adipate | 7 | 7 | 7 | - |

| **Autre composant** | | | | |
|---|---|---|---|---|
| Tocopherol | 0,007535 | 0,003 | - | - |
| **% total prémélange** | **58,57** | **49,5** | **39,5** | **32,5** |

Le tableau 8 résume les formulations des différents prémélanges comprenant chacun 19,5% en poids de la composition de filtres solaires dérivés de triazine.

**[Tableau 8]**

| | **% INCI** | | | | |
|---|---|---|---|---|---|
| **Nom INCI** | **Prém 5 (Invention)** | **Prém 6 (Hors invention)** | **Prém 7 (Hors invention)** | **Prém 8 (Hors inventio n)** | **Prém 9 (Hors invention)** |
| **Filtre solaire dérivé de triazine** | | | | | |
| Ethylhexyl triazone | 4,5 | 4,5 | 4,5 | 4,5 | 4,5 |
| Diethylhexyl butamido triazone | 9 | 9 | 9 | 9 | 9 |
| Bis-ethylhexyloxyphenol methoxyphenyl triazine | 6 | 6 | 6 | 6 | 6 |

| **Autre filtre** | | | | | |
|---|---|---|---|---|---|
| Diethylamino hydroxybenzoyl hexyl benzoate | 9 | 9 | 9 | 9 | 9 |
| Ethylhexyl salicylate | 4,5 | 4,5 | 4,5 | 4,5 | 4,5 |

| **Solubilisant** | | | | | |
|---|---|---|---|---|---|
| Diisopropyl sebacate | 13 | - | 13 | 13 | 13 |
| Dicaprylyl carbonate | 19,564129 | 19,564129 | 19,564129 | - | - |
| Propylheptyl caprylate | 9,95 | 9,95 | - | - | - |
| Dibutyl adipate | 8 | 8 | 8 | 8 | - |

| **Autre composant** | | | | | |
|---|---|---|---|---|---|
| Tocopherol | 0,010871 | 0,010871 | 0,005871 | - | - |
| **% total prémélange** | **83,57** | **70,57** | **73,57** | **54** | **46** |

Les prémélanges Prem 1 et Prem 5 contiennent 4 solubilisants selon l'invention. Les effets de la suppression d'1 ; 2 ou 3 solubilisants selon l'invention sur la stabilité des formules à 4°C ont été examinées pendant 2 mois en vérifiant l'aspect des formules (Prem 2 à 4 et 6 à 9).

### VI.3 - Résultats et conclusion

Les résultats sont résumés dans les tableaux 9 (formules 1 et 2 selon l'exemple I) et 10 (formule selon l'exemple II) ci-dessous.

**[Tableau 9]**

| **Stabilité** | **Prém 1** | **Prém 2** | **Prém 3** | **Prém 4** |
|---|---|---|---|---|
| **Stabilité à 4°C** | Stable à 2 mois | Stable à 2 mois | 7 jours : cristallisation | 7 jours : cristallisation |

**[Tableau 10]**

| **Stabilité** | **Prém 5** | **Prém 6** | **Prém 7** | **Prém 8** | **Prém 9** |
|---|---|---|---|---|---|
| **Stabilité à 4°C** | Stable à 2 mois | 7 jours : 1/3 piluliers - petits cristaux ; | 21 jours : Dépôt trouble ; | 7 jours : cristallisation | 7 jours : cristallisation |
| | | 21 jours : 2/3 piluliers - petits cristaux ; | | | |
| | | | 45 jours : cristallisation | | |
| | | 1 mois : 3/3 piluliers - cristaux | | | |

Les formules des exemples I et II contiennent trois triazines différents. Les formules 1 et 2 de l'exemple I contiennent 11,5% en poids de la composition de filtres solaires dérivé de triazine. Les résultats montrent qu'au moins 3 solubilisants selon l'invention (diisopropyl sebacate, dicaprylyl carbonate, dibutyl adipate - INCI) sont nécessaires pour éviter la recristallisation des filtres solaires dérivé de triazine à 4°C (prémélanges 1 à 4), pendant la période d'évaluation.

Les prémélanges 5 à 9, correspondants à la formulation de l'exemple II, contiennent 19,5% en poids de la composition de filtres solaires dérivé de triazine. Dans cette composition en haute teneur en filtres solaires dérivés de triazine, au moins 4 solubilisants selon l'invention (diisopropyl sebacate, dicaprylyl carbonate, dibutyl adipate, propylheptyl caprylate - INCI) sont nécessaires pour éviter la recristallisation des filtres solaires dérivé de triazine à 4°C pendant la période de l'évaluation.

La suppression du diisopropyl sebacate, du propylheptyl caprylate, ou la suppression simultanée des solubilisants correspondants aux désignations INCI dicaprylyl carbonate/propylheptyl caprylate et dicaprylyl carbonate/propylheptyl caprylate/dibutyl adipate entraine la précipitation des filtres solaires dérivé de triazine avec formation de cristaux (Prém 3, 4 et 6 à 9).

### REFERENCES BIBLIOGRAPHIQUES

Axelstad M., Boberg J., Hougaard K.S., Christiansen S., Jacobsen P.R., Mandrup K.R., Nellemann C., Lund S.P., Hass U. Effects of pre- and postnatal exposure to the UV-filter octyl methoxycinnamate (OMC) on the reproductive, auditory and neurological development of rat offspring. Toxicol. Appl. Pharmacol. 250 : 278-90 (2011).

Kinnberg K.L., Petersen G.I., Albrektsen M., Minghlani M., Awad S.M., Holbech B.F., Green J.W., Bjerregaard P., Holbech H.. Endocrine-disrupting effect of the ultraviolet filter benzophenone-3 in zebrafish, Danio rerio. Environ. Toxicol. Chem. 34 : 2833-40 (2015).

Ozáez, I., Morcillo, G., Martínez-Guitarte, J.L. Ultraviolet filters differentially impact the expression of key endocrine and stress genes in embryos and larvae of Chironomus riparius. Sci. Total Environ. 557-558 : 240-247 (2016).

Petersen, G., Rasmussen, D., Gustavson, K. Study on enhancing the Endocrine Disrupter priority list with a focus on low production volume chemicals Study on enhancing the Endocrine. Revised report to European Commission /DG Environment (2007).

Rehfeld A., et al. Organic Ultraviolet Filters Mimic the Action of Progestérone on Human Sperm and Interfère with Sperm Functions. FRI 105-133-Endocrine Disrupting Chemicals and Gene Régulation and Development (posters) (2016).

Schlumpf M., Cotton B., Conscience M., Haller V., Steinmann B., Lichtensteiger W. In vitro and in vivo estrogenicity of UV screens. Environ. Health Perspect. 109 : 239-44 (2001).

Schlumpf M., Durrer S., Faass O., Ehnes C., Fuetsch M., Gaille C., Henseler M., Hofkamp L., Maerkel K., Reolon S., Timms B., Tresguerres J.A., Lichtensteiger W. Developmental toxicity of UV filters and environmental exposure: a review. IntJAndrol. 31 : 144-51 (2008).

Szwarcfarb B., Carbone S., Reynoso R., Bollero G., Ponzo O., Moguilevsky J., Scacchi P. Octyl-Methoxycinnamate (OMC), an Ultraviolet (UV) Filter, Alters LHRH and Amino Acid Neurotransmitters Release from Hypothalamus of Immature Rats. Exp. Clin. Endocrinol. Diabètes 116 : 94-98 (2008).

Zucchi, S., Blüthgen, N., Ieronimo, A., Fent, K. The UV-absorber benzophenone-4 alters transcripts of genes involved in hormonal pathways in zebrafish (Danio rerio) eleuthero-embryos and adult males. Toxicol. Appl. Pharmacol. 250 : 137-46 (2011).

## Revendications

1. Composition comprenant :
- au moins les solubilisants correspondant aux désignations INCI suivantes : dibutyl adipate, dicaprylyl carbonate, diisopropyl sebacate et propylheptyl caprylate ; et
- au moins un filtre solaire dérivé de triazine représentant une quantité supérieure à 10% en poids total de la composition.

2. Composition selon la revendication 1, **caractérisée en ce qu'**au le moins un filtre solaire dérivé de triazine est choisi dans le groupe comprenant les filtres solaires dérivés de 1,3,5-triazines, de 1,2,4-triazines et de 1,2,3-triazines, avantageusement les filtres solaires dérivés de 1,3,5-triazines.

3. Composition selon l'une des revendications précédentes, **caractérisée en ce que** le au moins un filtre solaire dérivé de triazine est choisi dans le groupe comprenant les composés correspondant aux désignations INCI suivantes : bis ethylhexyloxyphenol methoxyphenyl triazine, diethylhexyl butamido triazone, tris-biphenyl triazine, ethylhexyl triazone, phenylene bis-diphenyltriazine et ethylhexyl bis-isopentylbenzoxazolylphenyl melamine.

4. Composition selon l'une des revendications précédentes, **caractérisée en ce qu'**elle comprend deux filtres solaires dérivés de triazine différents.

5. Composition selon l'une des revendications précédentes, **caractérisée en ce qu'**elle comprend trois filtres solaires dérivés de triazine différents.

6. Composition selon une quelconque des revendications précédentes, **caractérisée en ce qu'**elle comprend au moins les filtres solaires dérivés de triazine correspondant aux désignations INCI suivantes : bis ethylhexyloxyphenol methoxyphenyl triazine, diethylhexyl butamido triazone et ethylhexyl triazone.

7. Composition selon l'une des revendications précédentes, **caractérisée en ce que** le au moins un filtre solaire dérivé de triazine représente une quantité supérieure à 15% en poids total de la composition.

8. Composition selon l'une des revendications précédentes, **caractérisée en ce que** les solubilisants représentent entre 5% et 80% en poids total de la composition, avantageusement entre 10% et 70%, de préférence entre 15% et 60%.

9. Composition selon l'une des revendications précédentes, **caractérisée** en qu'elle comprend au moins un filtre solaire UV-A choisi dans le groupe comprenant les composés correspondant aux désignations INCI suivantes : butyl methoxydibenzoylmethane, diethylamino hydroxybenzoyl hexyl benzoate, bis-(diethylaminohydroxybenzoyl benzoyl) piperazine, disodium phenyl dibenzimidazole tetrasulfonate et leurs mélanges.

10. Composition selon l'une des revendications précédentes, **caractérisée** en qu'elle comprend au moins un écran minéral choisi dans le groupe comprenant les composés correspondant aux désignations INCI suivantes : zinc oxide, titanium dioxide et leurs mélanges.

11. Composition selon l'une des revendications précédentes, **caractérisée en ce que** la composition est exempte des filtres solaires correspondant aux désignations INCI suivantes : 4-methylbenzylidene camphor, benzophenone-2, benzophenone-3, ethylhexyl methoxycinnamate et octocrylene.

12. Composition selon l'une des revendications précédentes, **caractérisée en ce qu'**elle présente un SPF égal ou supérieur à 30, avantageusement égal ou supérieur à 50, de préférence égal ou supérieur à 70, voire égal ou supérieur à 100.

13. Composition selon l'une des revendications précédentes, pour son utilisation pour protéger la peau et/ou les muqueuses contre le rayonnement solaire ultraviolet.

14. Utilisation non-thérapeutique d'une composition selon l'une des revendications 1 à 12 pour protéger les phanères contre le rayonnement solaire ultraviolet.

## Patentansprüche

1. Zusammensetzung mit:
- mindestens den Solubilisierungsmitteln, die den folgenden INCI-Bezeichnungen entsprechen: Dibutyladipat, Dicaprylylcarbonat, Diisopropylsebacat und Propylheptylcaprylat; und
- mindestens einem von Triazin abgeleiteten Sonnenfilter, der eine Menge von mehr als 10 % des Gesamtgewichts der Zusammensetzung ausmacht.

2. Zusammensetzung nach Anspruch 1, **dadurch gekennzeichnet, dass** der mindestens eine von Triazin abgeleitete Sonnenschutzfilter aus der Gruppe ausgewählt ist, die Sonnenschutzfilter umfasst, die von 1,3,5-Triazinen, 1,2,4-Triazinen und 1,2,3-Triazinen abgeleitet sind, vorteilhafterweise Sonnenschutzfilter, die von 1,3,5-Triazinen abgeleitet sind.

3. Zusammensetzung nach einem der vorstehenden Ansprüche, **dadurch gekennzeichnet, dass** der mindestens eine von Triazin abgeleitete Sonnenfilter aus der Gruppe ausgewählt ist, die Verbindungen umfasst, die den folgenden INCI-Bezeichnungen entsprechen: Bis-ethylhexyloxyphenol Methoxyphenyltriazin, Diethylhexyl Butamido Triazon, Tris-Biphenyltriazin, Ethylhexyl Triazon, Phenylene bis-diphenyltriazine und Ethylhexyl bis-isopentylbenzoxazolylphenyl Melamine.

4. Zusammensetzung nach einem der vorstehenden Ansprüche, **dadurch gekennzeichnet, dass** sie zwei verschiedene, von Triazin abgeleitete Sonnenfilter umfasst.

5. Zusammensetzung nach einem der vorstehenden Ansprüche, **dadurch gekennzeichnet, dass** sie drei verschiedene, von Triazin abgeleitete Sonnenfilter umfasst.

6. Zusammensetzung nach einem der vorstehenden Ansprüche, **dadurch gekennzeichnet, dass** sie mindestens die von Triazin abgeleiteten Sonnenfilter umfasst, die den folgenden INCI-Bezeichnungen entsprechen: Bis-ethylhexyloxyphenol Methoxyphenyltriazin, Diethylhexyl Butamido Triazon und Ethylhexyl Triazon.

7. Zusammensetzung nach einem der vorstehenden Ansprüche, **dadurch gekennzeichnet, dass** der mindestens eine von Triazin abgeleitete Sonnenschutzfilter eine Menge von mehr als 15% des Gesamtgewichts der Zusammensetzung ausmacht.

8. Zusammensetzung nach einem der vorstehenden Ansprüche, **dadurch gekennzeichnet, dass** die Solubilisierungsmittel zwischen 5 % und 80 %, vorteilhafterweise zwischen 10 % und 70 %, bevorzugt zwischen 15 % und 60 %, des Gesamtgewichts der Zusammensetzung ausmachen.

9. Zusammensetzung nach einem der vorstehenden Ansprüche, **dadurch gekennzeichnet, dass** sie mindestens einen UV-A-Sonnenfilter umfasst, der aus der Gruppe ausgewählt ist, die aus Verbindungen besteht, die den folgenden INCI-Bezeichnungen entsprechen: Butylmethoxydibenzoylmethan, Diethylamino Hydroxybenzoyl Hexyl Benzoate, Bis-(Diethylaminohydroxybenzoyl Benzoyl) Piperazine, Disodium Phenyl Dibenzimidazole Tetrasulfonate und Mischungen davon.

10. Zusammensetzung nach einem der vorstehenden Ansprüche, **dadurch gekennzeichnet, dass** sie mindestens einen mineralischen Lichtschutzfilter umfasst, der aus der Gruppe ausgewählt ist, die aus Verbindungen besteht, die den folgenden INCI-Bezeichnungen entsprechen: Zinkoxid, Titandioxid und Mischungen davon.

11. Zusammensetzung nach einem der vorstehenden Ansprüche, **dadurch gekennzeichnet, dass** die Zusammensetzung frei von Sonnenschutzfiltern ist, die den folgenden INCI-Bezeichnungen entsprechen: 4-Methylbenzylidencampher, Benzophenon-2, Benzophenon-3, Ethylhexyl-Methoxycinnamate und Octocrylen.

12. Zusammensetzung nach einem der vorstehenden Ansprüche, **dadurch gekennzeichnet, dass** sie einen SPF von gleich oder größer 30, vorteilhafterweise gleich oder größer 50, bevorzugt gleich oder größer 70 oder sogar gleich oder größer 100 aufweist.

13. Zusammensetzung nach einem der vorstehenden Ansprüche zur Verwendung zum Schutz der Haut und/oder der Schleimhäute vor ultravioletter Sonnenstrahlung.

14. Nicht-therapeutische Verwendung einer Zusammensetzung nach einem der Ansprüche 1 bis 12 zum Schutz von Phaneren (Hautanhangsgebilden) vor ultravioletter Sonnenstrahlung.

## Claims

1. A composition comprising:
- at least the solubilizers corresponding to the following INCI designations: dibutyl adipate, dicaprylyl carbonate, diisopropyl sebacate and propylheptyl caprylate; and
- at least one sunscreen derived from triazine represents an amount greater than 10% of the total weight of the composition.

2. The composition according to claim 1, **characterized in that** at least one triazine-derived sunscreen is selected from the group comprising sunscreens derived from 1,3,5-triazines, 1,2,4-triazines and 1,2,3-triazines, advantageously sunscreens derived from 1,3,5-triazines.

3. The composition according to any one of the preceding claims, **characterized in that** the at least one triazine-derived sunscreen is chosen from the group comprising the compounds corresponding to the following INCI designations: bisethylhexyloxyphenol methoxyphenyl triazine, diethylhexyl butamido triazone, tris-biphenyl triazine, ethylhexyl triazone, phenylene bis-diphenyltriazine and ethylhexyl bis-isopentylbenzoxazolylphenyl melamine.

4. The composition according to any one of the preceding claims, **characterized in that** it comprises two different triazine-derived sunscreens.

5. The composition according to any one of the preceding claims, **characterized in that** it comprises three different triazine-derived sunscreens.

6. The composition according to any one of the preceding claims, **characterized in that** it comprises at least the triazine-derived sunscreens corresponding to the following INCI designations: bisethylhexyloxyphenol methoxyphenyl triazine, diethylhexyl butamido triazone and ethylhexyl triazone.

7. The composition according to any one of the preceding claims, **characterized in that** the at least one triazine-derived sunscreen represents an amount greater than 15% of the total weight of the composition.

8. The composition according to any one of the preceding claims, **characterized in that** the solubilizers represents between 5% and 80% of the total weight of the composition, advantageously between 10% and 70%, preferably between 15% and 60%.

9. The composition according to any one of the preceding claims, **characterized in that** it comprises at least one UV-A sunscreen chosen from the group comprising the compounds corresponding to the following INCI designations: butyl methoxydibenzoylmethane, diethylamino hydroxybenzoyl hexyl benzoate, bis-(diethylaminohydroxybenzoyl benzoyl) piperazine, disodium phenyl dibenzimidazole tetrasulfonate and mixtures thereof.

10. The composition according to any one of the preceding claims, **characterized in that** it comprises at least one inorganic screen chosen from the group comprising the compounds corresponding to the following INCI designations: zinc oxide, titanium dioxide and mixtures thereof.

11. The composition according to any one of the preceding claims, **characterized in that** the composition is free of sunscreens corresponding to the following INCI designations: 4-methylbenzylidene camphor, benzophenone-2, benzophenone-3, ethylhexyl methoxycinnamate and octocrylene.

12. The composition according to any one of the preceding claims, **characterized in that** it has an SPF equal to or greater than 30, advantageously equal to or greater than 50, preferably equal to or greater than 70, or even equal to or greater than 100.

13. The composition according to any one of the preceding claims, for its use for protecting skin and/or mucous membranes against ultraviolet solar radiation.

14. A non-therapeutic use of a composition according to any one of claims 1 to 12 for protecting dander against ultraviolet solar radiation.
